# EUROPEAN PATENT SPECIFICATION

(11) **EP 0 349 581 B1**
(45) Date of publication and mention of the grant of the patent: **03.01.1996**
(21) Application number: 88903086.2
(22) Date of filing: 11.03.1988
(51) Int. Cl.: G01K 13/00

(54) **DIAGNOSTIC TEMPERATURE PROBE**
TEMPERATURSONDE FÜR DIAGNOSE
SONDE THERMIQUE A USAGE DIAGNOSTIQUE

(30) Priority: 12.03.1987 US 25164
(43) Date of publication of application: 10.01.1990
(73) Proprietor: ABIOMED, INC., Danvers, MA 01923 (US)
(72) Inventor: KUNG, Robert, T., V., Andover, MA 01810 (US); MOUTAFIS, Timothy, E., Gloucester, MA 01930 (US); OCHS, Burt, D., North Andover, MA 01845 (US)
(74) Representative: Holdcroft, James Gerald, Dr.
(86) International application number: PCT/US88/00779
(87) International publication number: WO 88/06868

(56) References cited:
- FR-B- 2 448 714
- JP-A- 5 918 425
- US-A- 2 517 455
- US-A- 3 738 479
- US-A- 3 970 074
- US-A- 4 151 831
- US-A- 4 280 508
- US-A- 4 379 461
- US-A- 4 443 851
- US-A- 4 501 555
- US-A- 4 537 573
- See also references of WO8806868

## Description

The present invention relates to clinical periodontal instruments, and to the diagnosis of periodontal disease.

It is known in medicine generally to diagnose certain conditions of internal infection or inflammation by sensing the distribution of temperature over regions of the body, and identifying regions of abnormally high temperature as affected.

Some researchers have made measurements of periodontal pocket temperatures, which appear to vary greatly. Because of this large variation, one might expect a temperature-plotting approach to be of limited diagnostic utility in periodontics.

Owing to the physiological structure of the jaw, there are temperature gradients, from posterior to anterior regions, and from buccal to lingual sites on each side of the jaw. The presence of surface cooling due to breath air flow may further complicate the determination of even a normal temperature distribution. The combined effect of these sources of temperature variation is to mask from observation temperature anomalies which are less than approximately one-half degree Celsius. This would appear to limit the clinician's ability to identify with precision sites having temperature anomalies.

Journal of Oral Rehabilitation, 1978, Volume 5, pages 81-87 discloses a probe consisting of a 0.1 mm wire formed as a loop having a diameter of 2 mm. Its two metallic components are joined at the midpoint of the loop. From the loop the wire is extended as a 1 mm thick insulated, flexible cable which is supported by a handle.

Five points on either side of the mucogingival junction of 20 subjects were examined by means of the thermocouple probe. A mean difference amounting to about 2/3°C was found between the surface temperatures of the two structures.

An article titled "A Thermistor Probe for Measurement of Periodontal Temperature" by R T V Kung and J M Goodson discloses a thermistor probe designed and fabricated to investigate differences in periodontal pocket temperature as an indicator of disease activity. The probe was constructed with dimensions comparable to the Michigan "O" probe, and was designed with low thermal conductivity to avoid significant thermal perturbation of the measured region. In vitro response time was 0.2 sec., linear within 0.05 C in the range of 34 to 39C and constant for immersion depths of 3 mm or more.

Initial clinical investigations indicated that the standard deviation of periodontal pocket temperature measurements was 0.29C. Several normal temperature gradients were observed. A gradient of approximately 1C was observed from posterior (warmer) to anterior (cooler) periodontal pockets. Buccal sites were slightly warmer than lingual sites in the posterior regions and vice versa for the anterior teeth. On the average, there was a left/right symmetry in normal temperature gradients.

Correcting for expected normal temperature gradients, sites which exhibited disease activity by change in probeable attachment level had temperatures as much as 1C higher than sites which did not change in their probeable attachment. Pocket temperatures following treatment by local drug delivery decreased.

The authors conclude that measurement of periodontal pocket temperature differences could provide a simple and sensitive means for detection of periodontal disease activity.

US-A-4,501,555 discloses a periodontal probe comprising an elongate shaft terminating in a measuring tine having a plurality of spaced transverse grooves formed therein, the probe being formed of a material translucent to light. In use, the probe is mounted in a handle provided with a source of light to direct light along the length of the probe whereby the grooves formed in the tine appear as comparatively brightly illuminated marks.

The present invention overcomes the foregoing limitations of the prior art by providing apparatus as defined in claim 1 appended hereto.

A periodontal probe, embodying the invention and described hereinafter, has a handle and a probe finger extending from the handle to an L-shaped distal end. The distal end includes a probe stem portion adapted for insertion into a periodontal pocket and formed of a thermally insulative material, and a temperature sensing element mounted at the extreme end of the stem portion. Conductive leads extend from the sensing element, through the probe tip to the handle. In one embodiment, the temperature sensor is a thermistor which, together with the probe stem portion, is sealed within an epoxy outer skin. The tip tapers to approximately 0.41 mm (.016 inches) diameter, so that the thermal mass of the probe in the vicinity of the sensing element quickly attains the temperature of surrounding tissue while minimally perturbing that temperature. A prototype attains 0.10 °C accuracy with a 0.2 second response time.

In an illustrated prototype, the handle contains signal processing and temperature display units. Preferably, the probe tip includes graduations for indicating probe depth. Preferred signal processing circuitry includes sensor matching circuit elements, for adapting sensor elements having different characteristics to a common display driver.

With such a measurement instrument, it has been possible to accurately chart the variations in temperature of a normal mouth as a function of position.

In another preferred embodiment, the handle includes a control button for initiating a temperature measurement, and a microprocessor processes the sampled temperatures to detect diseased tissue. The processor includes means for processing temperature measurements, and means for determining a variation in temperature over a region of the mouth. A graphic display prompts entry of temperature measurements for different tissue sites, and the processor compares the measurements to a table of normal and anomalous jaw temperature distributions. The graphic display then indicates the health of the probed sites.

### Brief Description of the Drawings

These and other features of the invention will be understood from the following description of an illustrative embodiment, with reference to the drawings.
Figure 1 shows a perspective view of a probe according to the invention;
Figures 2A, 2B show details of the probe tip construction of one prototype probe similar to that of Figure 1;
Figure 3 is a schematic diagram of temperature sensing and display circuitry;
Figure 4 is a section through a probe having interchangeable sensor units;
Figure 5 shows a preferred clinical system embodying the invention;
Figure 6 shows another probe with a replaceable sensing tip;
Figure 7 shows the probe tip of Figure 6 at an earlier stage of manufacture and assembly;
Figure 8 shows a flow chart of a microprocessor-based clinical system as illustrated in Figure 5; and
Figure 9 shows a representative temperature analysis using a stored table of variations.

### Description

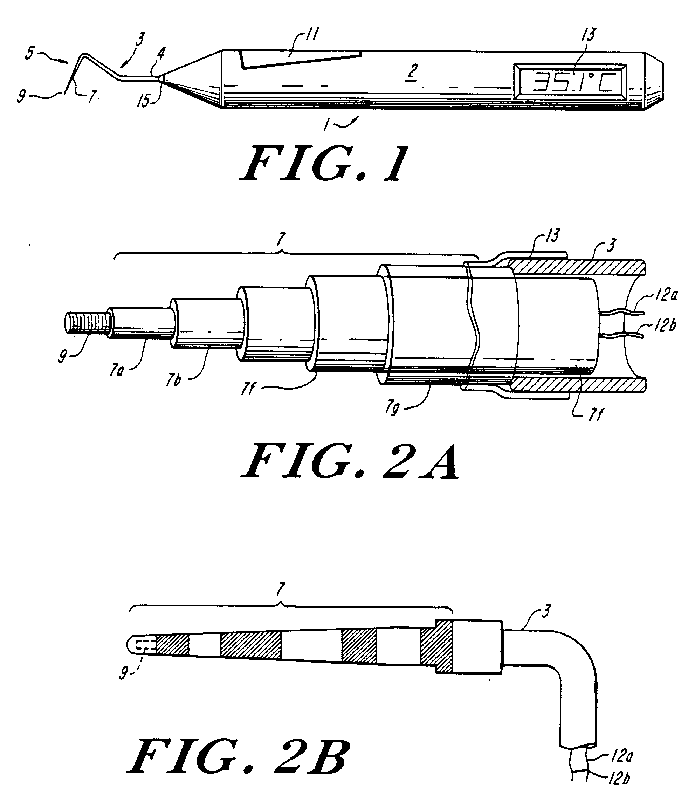

Figure 1 shows a perspective view of a prototype periodontal probe 1 according to the invention, having a handle portion 2 and a probe finger portion 3 extending from the handle. Finger portion 3 extends from a base end 4, which is firmly mounted in the handle, to a distal probe end 5 which has a general size and contour for probing a periodontal pocket.

At the distal end 5 of probe finger 3 a stem portion 7 having a length of approximately 1 cm. extends to a tip having a temperature sensing element 9 mounted thereat.

The overall length and shape of the probe 1 are substantially those of a Michigan "0" probe, and the base end 4 of the probe finger may be fabricated of metal. The handle 2, as discussed further below, houses signal conditioning and display circuitry for converting the temperature sensor output to a temperature display, and includes a power on switch 11 and three digit temperature display 13. Probe finger 3 attaches to handle 2 by a multi-contact electrical twist-connect fitting 15. Conductive leads (not shown) extend from the temperature sensing element, through the stem and finger to the fitting 15.

The probe stem is formed of a material having low thermal conductivity, such as a polyimide or epoxy compound, thus isolating the temperature sensor from the main body of the probe finger. Preferably, the probe stem material has a thermal conductivity less than 10⁻ watts/cm. °C. Applicant has found that the thermal conductivity of prior art probe fingers significantly perturbs the local temperature and limits the achievable accuracy of measurement and the response time.

Figure 2A shows a partial cutaway detail of construction of one probe tip having the desired strength and low thermal conductivity, employed in a prototype periodontal probe. As shown, finger portion 3 is formed from a 20 gauge thin-walled hypodermic needle, and stem portion 7 is made of a plurality of telescoping tubing segments 7a, 7b...7f, 7g formed of a commercially-available polyimide tube stock. The largest tube has a diameter of approximately 0.91 mm (.036 inches) and forms a shoulder against the end of the metal needle 3, and one or more of the smaller tube segments 7f...extends into needle 3. The telescoping tube segments form a stem which tapers from approximately 0.91 mm (.036 inches) down to approximately 0.36 (.014 inches) at the sensor end. Sensor 9 fits into the central bore of the smallest tube segment 7a.

During assembly, the conductive leads 12a, 12b of the sensor assembly 9 are passed through each tube as the tube segment is placed over the preceding segment. The wires are then passed through the bore of the needle 3, and cement is placed about the protruding portion of tube 7f and shoulder of tube 7g to secure the stem assembly into the needle. A thin plastic tube segment 13, which may be , for example, 0.05 mm (.002 inch) mylar is then heat-shrunk about the stem-finger joint to secure the components during further assembly.

Figure 2B shows the probe stem and tip assembly of Figure 2A after such fabrication. The telescoping tapered stem and sensor unit 7, 9 is potted to form an epoxy-sealed assembly. One suitable epoxy is the epoxy resin sold as Shell 828, with a Versamid 140 hardener. Following potting, Williams markings are painted on the sealed, tapered stem assembly to show depth of penetration of the sensor 9, which is indicated in phantom. As shown, the markings are graduated to one centimeter, although longer probes may be constructed.

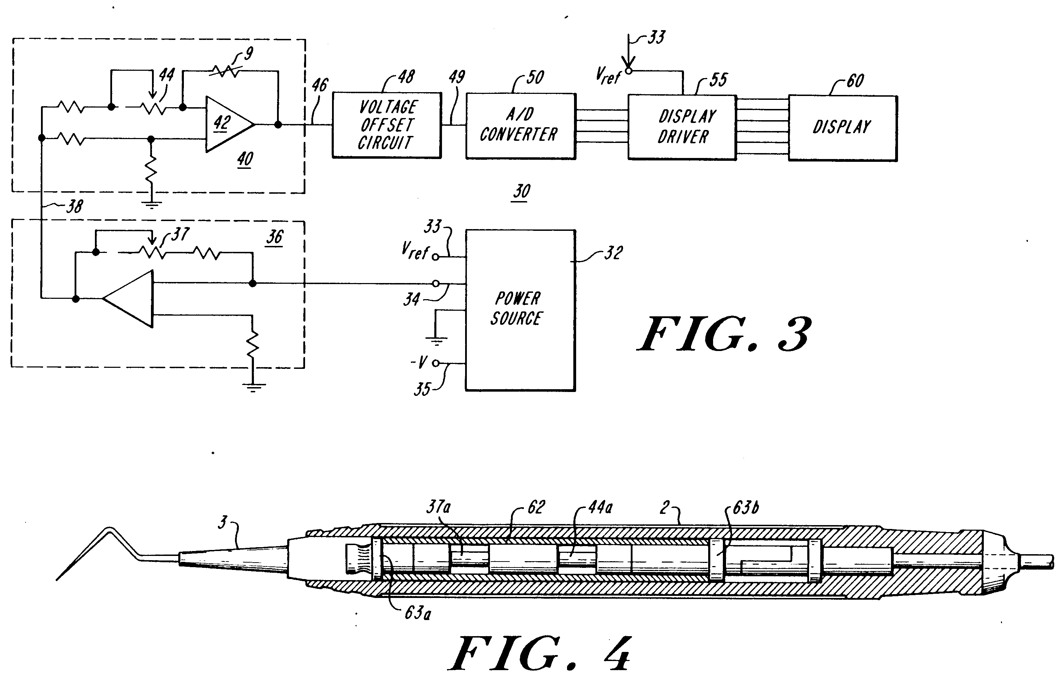

Figure 3 shows a block diagram of a circuit 30 used in a prototype embodiment of the probe. Circuit 30 includes a power supply 32, which provides a fixed reference voltage at output 33 and voltages at outputs 34, 35 from a battery source. Voltage outputs 33, 34, 35 are achieved by providing a zener diode shunt to achieve a fixed voltage drop, which is then passed to a center-grounded voltage-dividing network in a manner known in the art. The drive voltage from output 34 passes to a voltage setting section 36 including an op amp with a feedback potentiometer 37 for adjusting amplifier gain so as to provide a selected stable output voltage along line 38.

The voltage on line 38 passes to a temperature sensing circuit 40 in which a second op amp 42 having a thermistor temperature sensor 9 in a feedback loop is adjusted, via second potentiometer 44 in a resistance bridge, so that the circuit 40 produces a zero volt output on line 46 when the thermistor 9 is at thirty seven degrees Celsius.

The potentiometers 37, 44 are adjusted upon assembly, with potentiometer 37 adjusted in accordance with the gain characteristics of sensing circuit 40 -- e.g., to provide a voltage which increases inversely to the nominal resistance of the selected thermistor 9 -- and with potentiometer 44 set such that a zero volt signal appears on line 46 at the desired nominal 37 degree temperature. The voltage on line 46 then varies, in a substantially linear fashion, as the temperature of the thermistor changes.

Thermistor 9 has a resistance in the range of 30 - 60 kilohms, and draws a steady state current which is sufficiently low to not perturb the temperature readings by internal heat generation. The temperature-indicative voltage signal on line 46 passes to a voltage offset circuit 48, which increments the voltage on line 46 by a fixed voltage offset to produce an offset temperature-indicating signal on line 49. The signal on line 49 is digitized and scaled with respect to the reference line 33 by an analog to digital converter 50, and the scaled digitized signal passes to a display driver 55 which drives a display 60. In the embodiment shown in Figure 1, the display displays temperature readings in one-tenth degree increments.

In a preferred embodiment, the probe finger and tip assembly 3 as shown in Figure 1 is a removably interchangeable tip assembly which connects to the handle 2 by an electrical-contact type twist lock connector. In that embodiment, the potentiometers 37, 44, which in the prototype are used to match the nominal resistance and resistance gradient characteristics of the thermistor so as to produce an accurately scaled voltage-to-temperature reading, are replaced by one or more fixed resistance pairs 37a, 44a, 37b, 44b,..., each pair being matched to the characteristics of a single thermistor tip assembly.

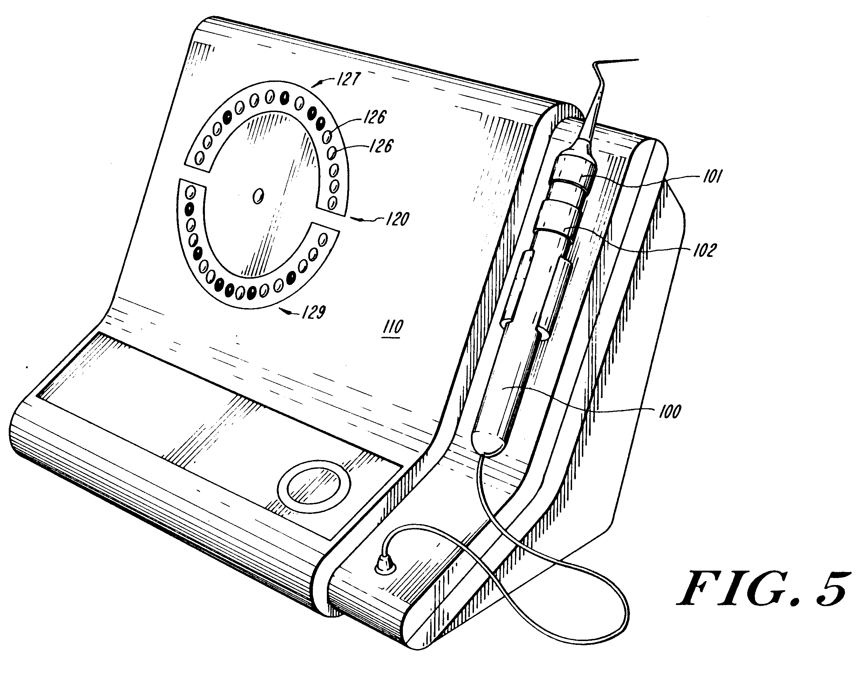

Figure 4 shows a section through such a preferred embodiment. As shown, handle 2 contains a resistor cartridge 62 holding one or more pairs of matched resistors 37a, 44a .... Cartridge 62 has a first end 63a adapted to engage the connector of probe tip 3, and a second end 63b which, upon engagement of the twist-locking probe tip connector places the correct pair of matched resistors into electrical contact with the voltage-setting and temperature-sensing circuitry. This allows replacement of tip assemblies, and permits the fitting of replacement temperature sensors, or temperature sensors adapted to detect finer temperature variations, or to accurately detect temperature variations centered about a different nominal center temperature.

Using a probe with a thermally isolated sensing end according to the invention accurate, repeatable measurements of periodontal pocket temperature have now been made. Analysis of such clinical measurements reveals that, with reference to the sublingual temperature of a subject, the periodontal temperature follows a predictable distribution which varies with the anatomic tooth location. Specifically, for a subject with healthy periodontal tissue, the temperatures of the periodontal pockets of upper jaw, or maxillary, teeth have been found to lie between (0.60) degrees and (2.53) degrees below the sublingual temperature as readings are taken of the third molar forward to the central incisor. Corresponding readings on the lower jaw, or mandibular, teeth vary between (0.06) and (1.19) degrees below the sublingual temperature. Generally, the temperature difference, referenced to sublingual, which is denoted ΔT herein, increases in magnitude from the back to the front of the jaw, from the lingual to buccal side of a given tooth, and between lower and corresponding upper jaw sites.

For each tooth location, a first temperature difference with respect to sublingual temperature, denoted ΔT'_{H} is a threshold indicative of healthy tissue, and a second, lesser magnitude temperature difference, denoted ΔT'_{D} indicates a higher temperature indicative of locally diseased tissue. Table 1 below shows a preliminary compilation of healthy and disease temperature difference thresholds used in a preferred diagnostic instrument to detect tissue health, as further described below.

**TABLE I**

| ΔT THRESHOLDS | | | |
|---|---|---|---|
| | TOOTH | HEALTHY THRESHOLD ΔT'_{H} | DISEASED THRESHOLD ΔT'_{D} |
| Maxillary Upper Jaw | 3rd Molar | -0.60 | -0.22 |
| | 2nd Molar | -0.60 | -0.22 |
| | 1st Molar | -1.08 | -0.39 |
| | 2nd Bicuspid | -1.64 | -0.81 |
| | 1st Bicuspid | -2.09 | -1.16 |
| | Canine | -2.33 | -1.32 |
| | Lateral Incisor | -2.50 | -1.31 |
| | Central Incisor | -2.53 | -1.47 |
| | | | |
| Mandibular Lower Jaw | 3rd Molar | -0.06 | +0.06 |
| | 2nd Molar | -0.06 | +0.06 |
| | 1st Molar | -0.47 | -0.15 |
| | 2nd Bicuspid | -0.87 | -0.47 |
| | 1st Bicuspid | -1.13 | -0.60 |
| | Canine | -1.17 | -0.73 |
| | Lateral Incisor | -1.16 | -0.77 |
| | Central Incisor | -1.19 | -0.83 |

According to another aspect of the invention, illustrated in Figure 5, a system includes a temperature sensing probe 100 and a processing unit 110 connected to the probe for processing temperature signals developed by the probe. Unit 110 includes a display 120 in the form of a map 125 of the jaw. Map 125 includes a plurality of light emitting diodes 126 (LEDs) with each diode position corresponding to one tooth location on the upper 127 or lower 129 jaw. A central diode 130 represents the sublingual region. As described more fully below, the LEDs are multi-color LEDs which may each be actuated to provide two, and preferably three, color indications. The handle of probe 100 includes two actuation buttons, 101, 102 denoted the "enter" and "advance" buttons, coupled to the processing unit as described below.

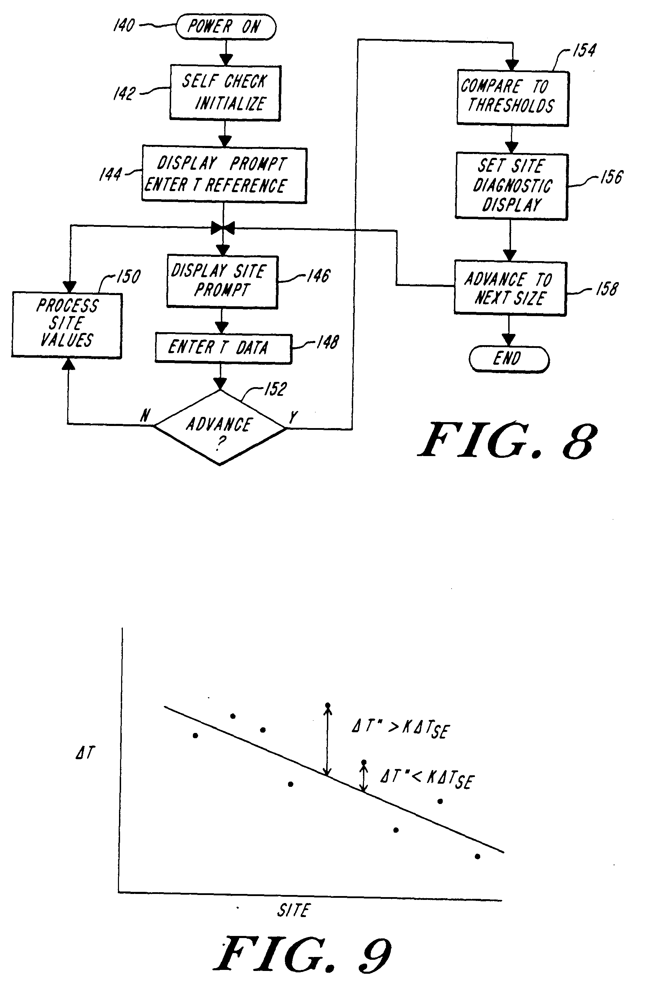

Operation of unit 110 is illustrated by the flowchart, Figure 9. Unit 110 includes a microprocessor having a ROM table memory which stores a set of temperature difference thresholds as shown in Table 1 above, and includes polling and arithmetical processing units for converting successive probe readings to temperature values, means for associating such readings with tooth locations, and means for deriving the measured ΔT value for a location and comparing it to the stored thresholds to determine when a periodontal pocket is diseased.

After turning on the power at step 140, the processor loads an instruction set, initializes its registers and performs a self check 142. The self check ascertains that all LEDs are operative, that probe leads are connected, and that the operating program is properly initialized.

At 144, the processor then actuates a display driver to prompt the operator to enter the reference site temperature. In the illustrated embodiment, the central diode 130 is intermittently actuated as a blinking green light to instruct the operator to enter the sublingual temperature by placing the probe tip below the subject's tongue and pressing the enter button 101 (Figure 5). Following entry of one or more sublingual temperature measurements, the advance button 102 is pressed, and the entered temperatures for that site are processed to determine an average sublingual temperature which is stored as a reference temperature against which subsequent periodontal temperature differences are determined.

The processor at 146 then connects the display driver to the LED of a next tooth location (e.g., the left upper third molar) to prompt the operator to place the probe at sites around that tooth, and polls the probe to determine when the "enter" and "advance" buttons are pressed. Each time the "enter" button 101 is pressed, a temperature reading is taken and processed at step 150. The invention contemplates operation in several processing modes, including a mode wherein successive temperature measurements at a single site or sites around a tooth are averaged to eliminate random measurement variations.

During data entry at a site, the LED associated with that site continues to blink green, indicating to the operator the present sampling location. Next at 152 the operator presses the advance button 102. This turns off the blinking green LED and initiates arithmetical processing wherein the processed site value is substracted from the sublingual reference temperature to determine ΔT for that site. At step 154, this ΔT value is compared to the stored table of threshold healthy and diseased temperature differences for the indicated jaw location. Based on this comparison, the sampled site is classified as healthy (H), diseased (D) or indeterminate (I). At step 156 the display for that site is actuated to display an H, D or I indication, and this display is thereafter maintained on the corresponding LED 126 as the processor advances its polling to the next tooth (left upper second molar, in this example) and repeats loop 160.

In the prototype embodiment, LED's 125 are two-color LEDs. Actuation with a first voltage signal drives a red display, and actuation with a second voltage signal drives a green display. Simultaneous actuation of red and green produces a yellow light. The red and green colors are used to indicate (D) and (H) states respectively, with yellow indicating indefinite diagnosis (I).

After the operator has performed measurements on one or more tooth locations by pressing the advance button to cycle through the successive jaw locations, the display continues to show the (D), (H) or (I) determinations for all sites tested. Repeat or replacement measurements, if desired, can be performed for one or more selected teeth by advancing to those teeth and entering new data. These diagnoses may then be checked off by the operator on a conventional printed jaw chart, or optionally may be printed out, together with actual temperature measurements if desired, for record purposes.

For the processing of measured temperatures, two basic operations are performed, designated mode 1 and mode 2. In mode 1, the periodontal site temperature T for a tooth is compared to the sublingual temperature Tₛ to develop the difference ΔT = T - Tₛ, which is typically less than zero. If ΔT < ΔT'_{H}, the stored "healthy threshold" for the tooth, the site is deemed healthy; if ΔT > ΔT'_{D} the "disease threshold", the site is deemed diseased. If ΔT lies between the two, i.e., ΔT'_{H} ≤ ΔT ≤ ΔT'_{D} the site is deemed indefinite and no definite diagnosis is given.

In mode 2 the temperatures are processed in a block. A number, e.g., nine site readings are queried on three adjacent teeth, with three sites (either all lingual or all buccal) on each tooth referenced to the sublingual temperature. A numerical processor performs a linear regression fit on the nine temperature measurements to obtain a best fit curve representing ΔT in the three-tooth region. Figure 9 shows such a graph ΔT in relation to nine sample points. The distances ΔT" of each temperature sample point from the graph is then formed, and is compared to a standard "error"of estimate" value ΔT_{SE} which has been compiled on a tooth-by-tooth basis from healthy subjects and is stored as a second table. The healthy/diseased diagnosis may be made based on whether ΔT" is less than or greater than K ΔT_{SE}, where K is a constant, which may be between one and two, the value of which is selected in accordance with a desired sensitivity of the determination. Other detection modes embodying different temperature comparisons are also contemplated.

### Returning now to the probe construction,

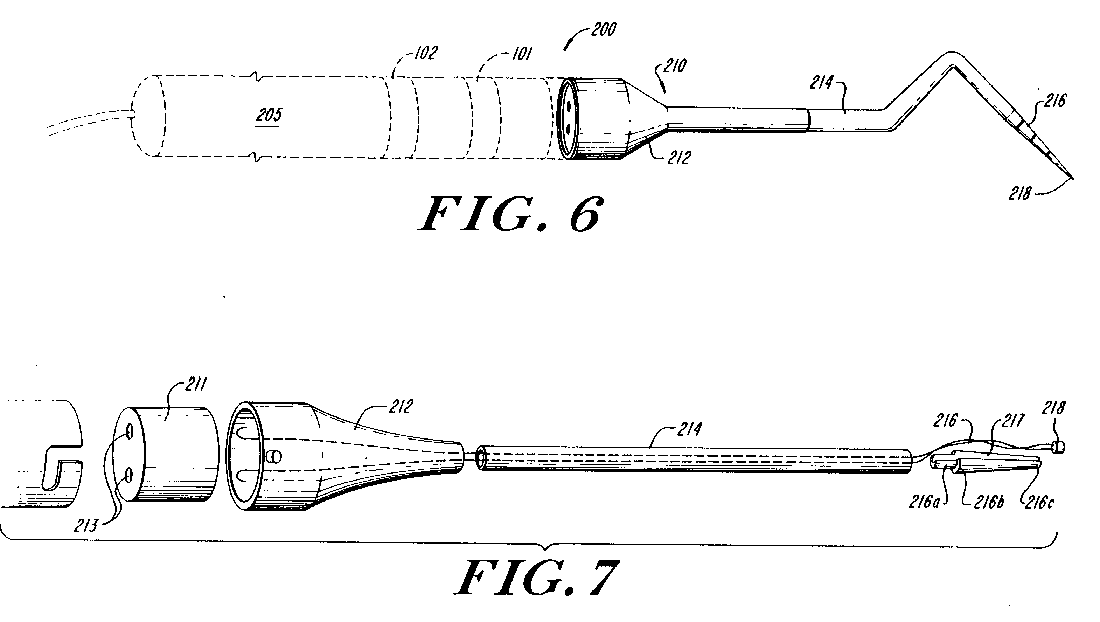

Figures 6 and 7 illustrate the structure and manufacture of a presently preferred probe 200 having the same overall size and shape as the probe 1 of Figure 1, and especially suitable for the system of Figure 5.

Probe 200 includes a handle 205, which may include circuitry as indicated in the probe of Figure 1 for converting a temperature sensor signal to a digital signal,and contains pushbutton "enter" and "advance" switches 101, 102 discussed above (not illustrated). A tip assembly 210 removably attaches to the handle and contains a temperature sensing element at the end thereof. Tip assembly 210 is fabricated as a disposable element of simplified construction. As shown, tip assembly 210 comprises four major portions, a shank 212, a tubular finger 214, a graduated tip 216 and an electrosensing temperature element 218, which extend successively away from the handle.

Figure 7 shows a disassembled perspective view, partly in phantom, of these elements at an early stage of assembly. Shank 212 is a molded plastic body, formed of Delrin, Nylon, ABS or similar tough polymer. Tubular finger 214 is formed of 20-gauge thin walled stainless steel stock. At the assembly stage finger 214 is a straight tube of approximately 3cm. length; upon completion of assembly the tube is bent to provide the characteristic periodontal probe Z-shape of Figures 1, 5. Tip or nosepiece 216 is formed of polysulfone or a similar strong polymer having low thermal conductivity, and has a slot 217 therein so that the conductive leads of sensor 218 may be conveniently channeled therethrough. After placement of the sensor 218 at the tip of piece 216 and threading the sensor leads through tube 214 and shank 212, the parts are cemented and pressed together, and tip 216 is dipped in epoxy, and cured, and the bands are printed thereon. Finally, a rubber bushing 211 with conductor receptacles 213 is fitted in shank 212 to provide a plug-like connection to the tip.

The tapered tip 216 has overall dimensions similar to those described for the multi-piece polymide tube embodiment of Figures 2A, 2B, tapering from about 0.89 mm (.035 inches) at its trunk 216b to approximately 0.41 mm (.016 inches) diameter at the sensor end 216c. A short stem portion 216a is dimensioned to press fit within tube 214 for assembly. Polysulfone material has been found to possess suitable strength for this part of these dimensions, and exhibits thermal conductivity of approximately 1 x 10⁻³ watts/cm. °C, with flexural modulus and flexural yield strength of 4 x 10⁵ and 1.5 x 10⁴ psi respectively. Other materials such as polyimides, polyamide/imides, and materials with similar properties may be used, to effectively provide a low mass thermally isolated sensor tip. Sensor element 218 is preferably a copper-constantin thermocouple.

It will be appreciated that the foregoing periodontal probes and systems have been described with reference to illustrated prototype embodiments and preferred variations thereof, but that the invention is not limited thereto. In other embodiments, different temperature sensing elements, different signal conditioning circuitry and different measurement processing may be employed, and different probe finger constructions and fittings are possible.

## Claims

1. Apparatus for performing a measurement in periodontal pockets in the mouth of a patient, for use with a probe assembly (1) including a shank (4, 212) which serves as a handle or as a connector for attachment to a handle (2, 205), and a tip portion extending from the shank, the tip portion comprising an elongated arm (3, 214) extending from the shank to a tip (7, 216) that tapers to a point for penetrating into a periodontal pocket, said apparatus further comprising :
- a temperature sensing element (9, 218) for taking a temperature measurement as said tip is positioned at a pocket location, and wherein said tapered tip portion is formed of a low thermal conductivity material for thermally isolating said sensing element from said probe, and
- a processor/display unit including
(i) a processor (110), and
(ii) an indicating display (120), the display (120) being arranged as a map (125) of tooth locations, and the processor actively associating the temperature measurements from the sensing element (9, 218) with locations of the map and processing the measurements to determine health of tissue at said locations.

2. Apparatus according to claim 1, wherein said tapered tip is formed of material having a thermal conductivity less than 10⁻ watt'cm.°C.

3. Apparatus according to claim 2, wherein said tip portion includes a material selected from an epoxy, a polysulfone, a polyamide or a polyamide/imide material.

4. Apparatus according to claim 2 or 3, wherein said sensing element (9, 218) is a thermocouple.

5. Apparatus according to any preceeding claim, wherein the processor (110) is in circuit communication with said temperature sensing element (9, 218) for processing a plurality of signals from the sensing element to determine a measurement.

6. Apparatus according to any preceding claim, wherein the processor display unit includes
a) means for actuating said display (120) to prompt a user to insert the probe assembly (1) at a location indicated on the map for taking a measurement thereat, and
b) means for determining as a function of the location whether the measurement is representative of a healthy or diseased tissue state.

7. Apparatus according to any preceding claim, wherein said processor (110) includes means for comparing a measured temperature of a tissue location to a stored reference temperature which is a function of the tissue location.

8. Apparatus according to any preceding claim, wherein said processor (110) includes a stored table of characteristic site temperature difference values, and also includes means for comparing a measured tissue site difference with a stored value for determining tissue site health.

9. Apparatus according to claim 8, wherein said processor (110) also includes means for comparing a measured variation with a characteristic tissue site difference for determining tissue site health.

10. Apparatus according to any preceding claim, wherein said display (120) includes a plurality of LEDs (126) arranged as a map of tooth sites and wherein said processor controls the states of said LEDs in accordance with entry of temperature measurement and diagnostic determinations of site temperature measurement.

11. Apparatus according to any preceding claim, wherein said indicating display (120) includes at least two different colored lights for each tooth location, and the processor (110) includes means for illuminating a light of a first color when said probe senses a healthy condition and a light of a second color when said probe senses an unhealthy condition.

12. Apparatus according to claim 11, wherein the display includes green and red lights respectively for indicating a healthy condition and an unhealthy condition.

13. Apparatus according to claim 11, wherein the processor (110) includes a stored table of characteristic site temperature values, and is operable to compare a measured tissue site temperature value with a table of stored characteristic site values to determine tissue site health.

14. Apparatus according to any preceding claim, wherein the processor (110) is operable to selectively illuminate a light (126) to prompt a user to probe a tooth location indicated on the map (125) and associates the temperature measurement performed by the probe with the indicated tooth location.

15. Apparatus according to any preceding claim, further comprising means for prompting a user to successively insert the tip portion (7.216) in a series of periodontal pockets to obtain a series of measurements, and wherein the processor is operable to analyze the series of temperature measurements in relation to the locations of said pockets to determine whether a measurement performed at each pocket is indicative of disease.

## Patentansprüche

1. Vorrichtung zur Durchführung einer Messung in periodontalen Taschen im Mund eines Patienten, zur Verwendung mit einer Sondenanordnung (1), die einen Schaft (4, 212), der als ein Handgriff oder als ein Verbinder zum Anbringen an einem Handgriff (2, 205) dient, und einen von dem Schaft ausgehenden Vorderendbereich aufweist, wobei der Vorderendbereich einen langgestreckten Schenkel (3, 214) aufweist, der von dem Schaft zu einem Vorderende (7, 216) verläuft, das sich zu einer Spitze zum Eindringen in eine periodontale Tasche verjüngt,
wobei die Vorrichtung ferner folgendes aufweist:
- ein Temperaturfühlerelement (9, 218) zur Durchführung einer Temperaturmessung, wenn das Vorderende am Ort einer Tasche positioniert ist, wobei der verjüngte Vorderendbereich aus einem Material mit geringer Wärmeleitfähigkeit geformt ist, um das Fühlerelement gegenüber der Sonde thermisch zu trennen, und
- eine Prozessor/Display-Einheit, die aufweist:
(i) einen Prozessor (110) und
(ii) ein Anzeigedisplay (120), wobei das Display (120) als eine Abbildung (125) von Zahnlokationen ausgelegt ist und der Prozessor die Temperaturmessungen des Fühlerelements (9, 218) Lokationen der Abbildung aktiv zuordnet und die Messungen verarbeitet, um den Gesundheitszustand von Gewebe an diesen Lokationen zu bestimmen.

2. Vorrichtung nach Anspruch 1, wobei das verjüngte Vorderende aus einem Material mit einer Wärmeleitfähigkeit von weniger als 10⁻ W/cm· °C geformt ist.

3. Vorrichtung nach Anspruch 2, wobei der Vorderendbereich ein Material aufweist, das aus einem Epoxid-, einem Polysulfon-, einem Polyamid- oder einem Polyamid/Imid-Material ausgewählt ist.

4. Vorrichtung nach Anspruch 2 oder 3, wobei das Fühlerelement (9, 218) ein Thermoelement ist.

5. Vorrichtung nach einem der vorhergehenden Ansprüche, wobei der Prozessor (110) in Leitungsverbindung mit dem Temperaturfühlerelement (9, 218) ist, um eine Vielzahl von Signalen des Fühlerelements zu verarbeiten und eine Messung festzulegen.

6. Vorrichtung nach einem der vorhergehenden Ansprüche, wobei die Prozessor/Display-Einheit folgendes aufweist:
a) eine Einrichtung zum Betätigen des Displays (120), um einen Benutzer zu veranlassen, die Sondenanordnung (1) an einer auf der Abbildung angezeigten Lokation einzuführen, um dort eine Messung vorzunehmen, und
b) eine Einrichtung, um als eine Funktion der Lokation festzustellen, ob die Messung für einen gesunden oder einen erkrankten Gewebezustand repräsentativ ist.

7. Vorrichtung nach einem der vorhergehenden Ansprüche, wobei der Prozessor (110) eine Einrichtung aufweist, um eine gemessene Temperatur einer Gewebelokation mit einer gespeicherten Referenztemperatur zu vergleichen, die eine Funktion der Gewebelokation ist.

8. Vorrichtung nach einem der vorhergehenden Ansprüche, wobei der Prozessor (110) eine gespeicherte Tabelle von charakteristischen Lokationstemperaturdifferenzwerten aufweist und außerdem eine Einrichtung aufweist, um eine gemessene Gewebelokationsdifferenz mit einem gespeicherten Wert zu vergleichen, um den Gesundheitszustand der Gewebelokation festzustellen.

9. Vorrichtung nach Anspruch 8, wobei der Prozessor (110) außerdem eine Einrichtung aufweist, um eine gemessene Abweichung mit einer charakteristischen Gewebelokationsdifferenz zu vergleichen, um den Gesundheitszustand der Gewebelokation festzustellen.

10. Vorrichtung nach einem der vorhergehenden Ansprüche, wobei das Display (120) eine Vielzahl von LEDs (126) aufweist, die als eine Abbildung von Zahnlokationen angeordnet sind, und wobei der Prozessor die Zustände dieser LEDs nach Maßgabe der Eingabe von Temperaturmessungen und diagnostischen Feststellungen der Lokationstemperaturmessung steuert.

11. Vorrichtung nach einem der vorhergehenden Ansprüche, wobei das Anzeigedisplay (120) wenigstens zwei verschiedenfarbige Lichter für jede Zahnlokation aufweist und der Prozessor (110) Mittel aufweist, um ein Licht einer ersten Farbe leuchten zu lassen, wenn die Sonde einen gesunden Zustand erfaßt, und ein Licht einer zweiten Farbe leuchten zu lassen, wenn die Sonde einen erkrankten Zustand erfaßt.

12. Vorrichtung nach Anspruch 11, wobei das Display grüne und rote Lichter aufweist, um einen gesunden Zustand bzw. einen erkrankten Zustand anzuzeigen.

13. Vorrichtung nach Anspruch 11, wobei der Prozessor (110) eine gespeicherte Tabelle von charakteristischen Lokationstemperaturwerten enthält und in der Lage ist, einen gemessenen Gewebelokations-Temperaturwert mit einer Tabelle von gespeicherten charakteristischen Lokationswerten zu vergleichen, um den Gesundheitszustand der Gewebelokation festzustellen.

14. Vorrichtung nach einem der vorhergehenden Ansprüche, wobei der Prozessor (110) in der Lage, ist selektiv ein Licht (126) aufleuchten zu lassen, um einen Benutzer zu veranlassen, eine auf der Abbildung (125) angezeigte Zahnlokation mit der Sonde zu untersuchen, und die von der Sonde durchgeführte Temperaturmessung der angezeigten Zahnlokation zuordnet.

15. Vorrichtung nach einem der vorhergehenden Ansprüche, die ferner Mittel aufweist, um einen Benutzer zu veranlassen, den Vorderendbereich (7, 216) nacheinander in eine Serie von periodontalen Taschen einzuführen, um eine Serie von Messungen zu erhalten, und wobei der Prozessor in der Lage ist, die Serie von Temperaturmessungen in bezug auf die Lokationen der Taschen zu analysieren und festzustellen, ob eine an jeder Tasche durchgeführte Messung für eine Erkrankung bezeichnend ist.

## Revendications

1. Appareil pour effectuer une mesure dans des cavités parodontales dans la bouche d'un patient, pour une utilisation avec un ensemble de sonde (1) comprenant un embout (4, 212) qui sert de poignée ou de connecteur pour une fixation à une poignée (2, 205), et une portion de pointe s'étendant à partir de l'embout, la portion de pointe comprenant un bras allongé (3, 214) s'étendant depuis l'embout vers une pointe (7, 216) qui s'amincit en un point pour pénétrer dans une cavité parodontale,
ledit appareil comprenant en outre
- un élément de détection de température (9, 218) pour effectuer une mesure de température quand ladite pointe est positionnée à un emplacement de cavité, et dans lequel ladite portion amincie de pointe est formée d'un matériau de conductibilité thermique faible pour isoler thermiquement ledit élément de détection de ladite sonde, et
- un module de traitement et d'affichage comprenant
(i) un processeur (110) et
(ii) un afficheur d'indication (120), l'afficheur (120) étant agencé comme une carte (125) des emplacements de dent et le processeur associant activement les mesures de température provenant de l'élément de détection (9, 218) à des emplacements de la carte et traitant les mesures pour déterminer l'état de santé des tissus auxdits emplacements.

2. Appareil selon la revendication 1, dans lequel ladite pointe amincie est formée d'un matériau ayant une conductibilité thermique inférieure à 10⁻ watt/cm ° C.

3. Appareil selon la revendication 2, dans lequel ladite portion en forme de pointe comprend un matériau sélectionné parmi un époxy, un polysulfone, un polyamide ou un matériau en polyamideimide.

4. Appareil selon la revendication 2 ou 3, dans lequel ledit élément de détection (9, 218) est un thermocouple.

5. Appareil selon l'une quelconque des revendications précédentes, dans lequel le processeur (110) est en communication de circuit avec ledit élément de détection de température (9, 218) pour traiter une pluralité de signaux provenant de l'élément de détection afin de déterminer une mesure.

6. Appareil selon l'une quelconque des revendications précédentes, dans lequel le module de traitement et d'affichage comprend :
a) des moyens pour activer ledit afficheur (120) pour inviter un utilisateur à insérer l'ensemble de sonde (1) en un emplacement indiqué sur la carte afin d'y effectuer une mesure, et
b) des moyens pour déterminer en fonction de l'emplacement si la mesure représente une état sain ou malade de tissu.

7. Appareil selon l'une quelconque des revendications précédentes, dans lequel ledit processeur (110) comprend des moyens pour comparer une température mesurée d'un emplacement de tissu à une température stockée de référence qui est une fonction de l'emplacement du tissu.

8. Appareil selon l'une quelconque des revendications précédentes, dans lequel ledit processeur (110) comprend une table stockée de valeurs caractéristiques de différence de température de sites, et comprend aussi des moyens pour comparer une différence mesurée de site de tissu à une valeur stockée, afin de déterminer l'état de santé du tissu.

9. Appareil selon la revendication 8, dans lequel ledit processeur (110) comprend des moyens pour comparer un écart mesuré avec des différences caractéristiques de site de tissu afin de déterminer l'état de santé du tissu.

10. Appareil selon l'une quelconque des revendications précédentes, dans lequel ledit afficheur (120) comprend une pluralité de diodes électroluminescentes (126) disposées comme une carte de sites de dent et dans lequel ledit processeur commande les états desdites diodes électroluminescentes en fonction d'une entrée de mesure de température et des déterminations de diagnostic de mesure de température de site.

11. Appareil selon l'une quelconque des revendications précédentes, dans lequel ledit afficheur d'indication (120) comprend au moins deux lumières de couleurs différentes pour chaque emplacement de dent, et le processeur (110) comprend des moyens pour allumer une lumière d'une première couleur quand ladite sonde détecte une condition saine, et une lumière d'une deuxième couleur quand ladite sonde détecte une condition de maladie.

12. Appareil selon la revendication 11, dans lequel l'afficheur comprend des lumières verte et rouge pour indiquer respectivement une condition saine et une condition de maladie.

13. Appareil selon la revendication 11, dans lequel le processeur (110) comprend une table stockée de valeurs caractéristiques de température de site, et peut fonctionner pour comparer une valeur mesurée de température de site avec une table de valeurs stockées caractéristiques de température de site pour déterminer l'état de santé d'un tissu.

14. Appareil selon l'une quelconque des revendications précédentes, dans lequel le processeur (110) peut fonctionner pour allumer sélectivement une lumière (126) afin d'inviter un utilisateur à sonder un emplacement de dent indiqué sur la carte (125) et associe la mesure de température exécutée par la sonde à l'emplacement indiqué de dent.

15. Appareil selon l'une quelconque des revendications précédentes, comprenant en outre des moyens pour inviter un utilisateur à insérer la portion de pointe (7, 216) dans une série de cavités parodontales afin d'obtenir une série de mesures, et dans lequel le processeur peut fonctionner pour analyser la série de mesures de température en liaison avec les emplacements desdites cavités pour déterminer si une mesure effectuée à chaque cavité indique une maladie.
